# EUROPEAN PATENT SPECIFICATION

(11) **EP 3 050 567 B1**
(45) Date of publication and mention of the grant of the patent: **04.11.2020**
(21) Application number: 13894637.1
(22) Date of filing: 26.09.2013
(51) Int. Cl.: A61K 31/52, A61P 29/00, A61P 3/10, A61P 25/28, A61P 17/02, A61P 39/06, A61P 35/00, A61P 3/04, A61P 3/06, A61P 3/00, A61P 43/00

(54) **COMPOUND FOR ACTIVATING AMPK AND USES THEREOF**
VERBINDUNG ZUR AKTIVIERUNG VON AMPK UND VERWENDUNGEN DAVON
COMPOSÉ SERVANT À L'ACTIVATION DE L'AMPK ET SES UTILISATIONS

(43) Date of publication of application: 03.08.2016
(60) Divisional application: 20194630.8
(73) Proprietor: Energenesis Biomedical Co., Ltd., New Taipei City 235 (TW)
(72) Inventor: CHIU, Jen-Yi, New Taipei 235 (TW); CHEN, Han-Min, New Taipei 235 (TW); KUO, Cheng-Yi, New Taipei 235 (TW); LIN, Jiun-Tsai, New Taipei 235 (CN); HUANG, Chun-Fang, New Taipei 235 (CN)
(74) Representative: Epping - Hermann - Fischer
(86) International application number: PCT/CN2013/084294
(87) International publication number: WO 2015/042821

(56) References cited:
- EP-A1- 1 302 196
- EP-A1- 1 665 940
- WO-A1-93/03734
- WO-A1-99/37151
- WO-A1-2009/014642
- WO-A1-2010/002492
- CN-A- 101 433 540
- CN-A- 101 744 191
- CN-A- 102 406 649
- RO-A2- 60 784
- US-A1- 2009 131 537
- US-A1- 2013 116 215
- JIANG, JIYANG ET AL.: 'The Antiinflammatory Effects of an Adenosine' CHINESE PHARMACOLOGICAL BULLETIN vol. 14, 30 October 1998, pages 79 - 82, XP008181896
- CAI, XIAOPIN ET AL.: 'The Clinical Applications and Prospects of Purinergic (P2Y) Receptor Agonist in Diabetes Treatment' JOURNAL OF PRACTICAL DIABETOLOGY vol. 3, no. 6, 31 December 2007, pages 10 - 11, XP008181906
- DAI, SHUANGSHUANG ET AL.: 'Different Effects of Adenosine A2A Receptors in the Models of Traumatic Brain Injury and Peripheral Tissue Injury' ACTA PHYSIOLOGICA SINICA vol. 60, no. 2, 25 April 2008, pages 254 - 258, XP008181892

## Description

### Technical Field

The present disclosure relates to adenine which is useful to activate AMPK (AMP-activated protein kinase) and the use of the compound in the prevention or treatment of conditions or disease.

### Background

AMPK is a cellular energy sensor and a responder to energy demand. AMPK is a heterotrimer composed of catalytic α subunit and regulatory β, γ subunits. All these subunits are highly conserved in eukaryotes. The activation of AMPK is through phosphorylation on the conserved 172^{th}-threonine residue of α subunit by upstream kinases such as LKB1, Ca2+/Calmodulin dependent kinase, and TAK1. High AMP/ATP ratio caused by physiological or pathological stress activates AMPK. Upon activation, AMPK activates catabolic pathway and inhibits anabolism which in term restores cellular energy balance by decreasing ATP consumption and promoting ATP generation.

As a regulator of energy homeostasis, AMPK has been suggested to be a potential drug target for metabolic syndromes including type II diabetes, cardio-vascular disease, and fatty liver disease. Many of the metabolic syndromes are linked to insulin resistance. Insulin resistance is a pathological condition in which cells fail to respond to insulin thus excess glucose in the blood stream cannot be removed into skeletal muscle or fat tissue. The activation of AMPK increases protein level of GLUT4, a glucose transporter, via transcriptional regulation and induces GLUT4 translocation to the plasma membrane in muscle cells in an insulin independent manner resulting in increases in the rate of cellular glucose uptake. Activation of AMPK also inhibits fatty acids and cholesterol synthesis via suppressing acetyl-CoA carboxylase and HMG-CoA reductase, respectively. In addition, activation of AMPK leads to inhibition of several transcription factors, including SREBP-1c, ChREBP and HNF-4a, and down-regulates the expression of enzymes which are mainly involved in fatty acid synthesis and gluconeogenesis. These findings support the idea that AMPK is a target of choice in the treatment of metabolic syndrome, in particular, diabetes.

In addition to the regulation of energy homeostasis, AMPK has been implicated in modulating several cellular mechanisms including inflammation, cell growth, apoptosis, autophagy, senescence and differentiation. Extensive studies demonstrate AMPK is a repressor of inflammation. Activation of AMPK can inhibit inflammation via suppressing NF-κB signaling. NF-κB signaling is the principle pathway that activates innate and adaptive immunity. The activation of AMPK can inhibits NF-κB transcriptional activity indirectly via stimulating SIRT1, Forkhead box O (FoxO) family or peroxisome proliferator-activated receptor co-activator 1α (PGC1α). Several groups also demonstrate that activation of AMPK suppresses protein expression of cyclooxygenase-2 (COX-2). COX-2 is an inducible enzyme which controlled by pro-inflammatory cytokines and growth factors. COX-2 converts arachidonic acid into prostaglandin which results in inflammation and pain. Inhibition of COX-2 activity or expression has been linked to anti-inflammation.

Several AMPK activators have been demonstrated to possess antiinflammatory function in vivo. For example, 5-aminoimidazole-4-carboxamide ribonucleoside (AICAR) has been shown to ameliorate acute and relapsing colitis mouse model induced by 2,4,6-trinitrobenzene sulfonic acid (TNBS) or dextran sulfate sodium. AICAR treated mice showed reduced body weight loss and significant attenuation of inflammation. AICAR also showed therapeutic effects in treating experimental autoimmune encephalomyelitis (EAE), an animal model of multiple sclerosis and decreases severity of LPS-induced lung injury in mice.

Dysregulation of cellular signaling pathway can lead to abnormal cell growth and ultimately, cancer. The mammalian target of rapamycin (mTOR) is a serine/threonine kinase which regulates cell proliferation and autophagy. The activity of mTOR signaling pathway is dys-regulated in many different cancers and therefore mTOR inhibitors are considered as potential drugs for cancer therapy. There are extensive studies demonstrate that AMPK phosphorylates tuberous sclerosis complex 2 (TSC2) and Raptor to inhibit mTOR pathway. A variety of AMPK activators including AICAR, metformin, phenformin has also been demonstrated suppressed mTOR signaling and inhibited cancer cell growth. In addition, activation of AMPK induces autophagy via suppressing mTORC1 activity. Due to the inhibition of mTORC1 by AMPK, phosphorylation of Ulk1 on Ser⁷⁵⁷ is decreased and subsequently Ulk1 can be phosphorylated by AMPK on Ser³¹⁷ and Ser⁷⁷⁷. The AMPK-phosphorylated Ulk1 is active and then initiates autophagy.

Base on above mentioned, AMPK has been suggested as a good target in many human diseases or pathological conditions including inflammatory disease, wound healing, neurodegeneration, cancer, oxidative stress and cardiovascular disease. In fact, AMPK activators have been applied for clinical trials in at least 24 disease categories including bacterial and fungal diseases, behaviors and mental disorders, blood and lymph conditions, cancers and other Neoplasms, digestive system diseases, diseases and abnormalities at or before birth, ear, nose, and throat diseases, eye diseases, gland and hormone related diseases, heart and blood diseases, immune system diseases, mouth and tooth diseases, muscle, bone, and cartilage diseases, nervous system diseases, nutritional and metabolic diseases, occupational diseases, parasitic diseases, respiratory tract diseases, skin and connective tissue diseases, wound healing and so on.

WO 93/03734 A1, RO 60784 A2, WO 2010/002492 A1, EP 1302196 A1, US 2013/116215 A1, EP 1665940 A1, US 2009/131537 A1, WO 99/37151 A1, WO 2009/014642 A2, CN 102406649 A, and CN 101744191 A *relate to compositions comprising adenine or an analogue therof and its use in the prevention or treatment of a number of illnesses.*

### Summary

The present disclosure provides a composition for use in preventing scar formation during wound healing according to claim 1 and a composition for use in enhancing wound healing according to claim 2.

### Detailed Description

The inventors have discovered that adenine is a novel AMPK activator and has various biological functions. In recent years, the activation of AMPK has been shown to be beneficial in the prevention and the treatment of diseases such as pre-diabetes, insulin resistance, type 2 diabetes, metabolic syndrome, obesity, inflammation, Alzheimer's disease, cancer, oxidative stress and cardiovascular disease as well as enhancing wound healing. The inventors contemplate that such effects can be attributed to the activation of AMPK which result in but not limited to the reduction of COX-2, ROS production and increase of glucose uptake.

### Contemplated Indications

Base on the inventor's findings (see examples below), it's contemplated that adenine can be used as a therapeutic agent for various conditions or diseases via activating AMPK. The following provides exemplary guidance and evidence on contemplated indications.

### Reference Example: Adenine in the Treatment of Hyperglycemia, Pre-diabetes, Insulin resistance, and Type 2 diabetes

It has recently been reported that AMPK activators including metformin, A769662, AICAR reduced plasma glucose in diabetic or obesity mice models. In the present disclosure, 1 µM-600 µM of adenine significantly increased glucose uptake of C2C12 muscle cells (Table 2). To further evaluate the effects of adenine on the modulation of plasma glucose level, the high-fat diet-fed mice were served as a type 2 diabetes animal model. Chronic treatment of high-fat diet-fed mice with adenine significantly reduced plasma glucose by more than 30% and decreased plasma triacylglycerides by more than 35% compared to the control mice. A more-than-15% decrease in body weight was also observed (example 3). As used herein, "hyperglycemia" refers to physiological condition characterized by blood sugar higher than 126 mg/dL. As used herein, "pre-diabetes" refers to a physiological condition characterized by a fasting blood sugar higher than 100 mg/dL but below than 140 mg/dL. As used herein, "insulin resistance" refers to a physiological condition in which whole body or tissues including liver, skeletal muscle, adipose tissue fail to response to insulin. As used herein, "type 2 diabetes" also refers to noninsulin-dependent diabetes mellitus (NIDDM) or adult-onset diabetes. It refers to a metabolic disorder caused by insufficient insulin production or insulin resistance which often manifested by a fasting glucose higher than 140 mg/dL. According to the examples, adenine was found to accelerate glucose uptake, therefore was suggested as a useful treatment to the conditions or diseases which associated with high blood glucose.

### Reference Example: Adenine in the Treatment of Inflammation

Various AMPK activators have been demonstrated possess antiinflammatory function *in vivo.* For example, daily treatment of 5-aminoimidazole-4-carboxamide ribonucleoside (AICAR) in 2,4,6-trinitrobenzene sulfonic acid (TNBS) or dextran sulfate sodium-treated mice ameliorates acute and relapsing colitis by reduced body weight loss and significant attenuation inflammation. Treatment of AICAR had therapeutic effects in experimental autoimmune encephalomyelitis (EAE), an animal model of multiple sclerosis. Treatment of AICAR to mice decreased severity of LPS-induced lung injury. In the present disclosure, adenine inhibited LPS-induced inflammation in vitro: under LPS stimulation, the secretion level of pro-inflammatory cytokines including TNFα, IL-1β and IL-6 were significantly reduced in adenine treated macrophages compared to control cells. Adenine also decreased COX-2 expression which was induced by LPS in human macrophages (example 4). In TNBS-induced inflammatory bowel disease (IBD) mice model chronic treatment with adenine significantly reduced pro-inflammatory cytokines including TNF, INFγ and IL-17 in colon compare to control mice and rescued body weight loss in these mice (example 5).

As used herein, "pro-inflammatory cytokines" refers to cytokines which promote systemic inflammation. "Inflammatory diseases" used herein refers to diseases associate with inflammation including but not limited to ankylosing spondylitis, arthritis (osteoarthritis, rheumatoid arthritis, psoriatic arthritis), asthma, atherosclerosis, Crohn's disease, colitis, dermatitis, diverticulitis, fibromyalgia, hepatitis, irritable bowel syndrome, systemic lupus erythematous, nephritis, Alzheimer's disease, Parkinson's disease and ulcerative colitis. Recently, several reports demonstrate that AMPK is an upstream regulator of COX-2 and suppresses COX-2 protein expression. The same with previous findings, the inventors found that a novel AMPK activator, adenine can also suppress COX-2 expression which suggest adenine may be a useful compound to inhibit COX-2 mediated inflammation. According to the present disclosure, adenine was found to be able to inhibit inflammation, therefore was suggested as a useful treatment to the conditions or diseases which associated with inflammation.

### Adenine in Wound Healing and Scar Formation

AMPK has been suggested to promote cell motility and enhance wound healing in cultured cells. An AMPK activator, resveratrol, has been found to enhance incisional wound healing. In addition to closing the wound, reducing scar formation during the healing process has been a preferred goal in modern medicament. Neonatal wound healing, unlike adult wound healing, does not accompany scar formation. The difference is in Cox-2 activation. In adult wound healing, COX-2 activity will be elevated (by TGF-beta), resulting in the increased production of prostaglandin at wound sites. Prostaglandin promotes fibroblast growth and collagen formation, two factors that lead to scar formation. Hence, inhibition of COX-2 activities has been considered as effective treatment in preventing scar formation. In the present disclosure, adenine inhibited human fibroblast cell growth (example 8) and reduce COX-2 expression. Using animal model, topical treatment of adenine at wound site enhanced not only wound closure but also reduced scar formation (example 9). According to above data, topical administration of adenine is useful to enhance would healing and prevent scar formation.

### Reference Example: Neurodegeneration

Defects in several different cellular mechanisms has been linked to neurodegeneration, including inflammation, intracellular trafficking, and autophagy. Autophagy functions to remove dysfunctional organelles or protein aggregates in the cell and play a crucial part in maintaining cellular homeostasis. Pathogenesis of many neurodegenerative diseases involves the presence of intracellular or extracellular protein aggregate deposits. Removal of these protein aggregates has been shown to ameliorate the progression of these diseases. Impaired autophagy pathway or removal of proteins responsible for autophagy has been linked to neurodegeneration. AMPK activation has been shown to facilitate autophagy pathway. Therefore, promotion of autophagy pathways via activation of AMPK may be a useful strategy to prevent or control neurodegeneration. AMPK activators have been shown to decrease amyloid deposition via autophagy pathway. Daily resveratrol administration increases life span in Alzheimer's disease (AD) mice models. Another AMPK activator, curcumin, has also been demonstrated as a potential drug for AD therapy. In the present disclosure, the inventors found that adenine significantly enhanced autophagy activity and reduced Aβ accumulation in a dose-dependent manner in Neuro2A cells and improved cognitive function in AD mice model (example 6 and 7). According to these findings, adenine can be useful in the treatment of neurodegenerative diseases.

As used herein, "neurodegeneration" refers to the condition which is progressive loss of structure or function of neurons. Neurodegenerative disease is a result of neurodegenerative processes including Alzheimer's disease, Parkinson's disease (PD), Huntington's disease (HD), amyotrophic lateral sclerosis (ALS), Spinocerebellar ataxia (SCA), Spinal muscular atrophy (SMA), etc.

### Reference Example: ROS Associated Diseases

Reactive oxygen species (ROS) including superoxide radicals, hydroxyl redical and hydrogen peroxide are continuously produced in tissues. A variety of diseases have been associated with excessive ROS including NARP (neurogenic muscle weakness, ataxia and retinitis pigmentosa), MELAS (mitochondrial encephalomyopathy, lactic acidosis, and stroke-like episodes), MERRF (myoclonic epilepsy and ragged-red fibers), LHON (Leber hereditary optic neuropathy), and KSS (Kearns-Sayre syndrome, ophthalmoplegia, ataxia, retinitis pigmentosa, cardiac conduction defect and elevated cerebrospinal fluid protein), Parkinson disease (PD), Alzheimer's disease (AD), amyotrophic lateral sclerosis (ALS), Huntington's disease (HD), and Friedreich's ataxia (FA) and aging. Numerous reports demonstrate that AMPK activator, AICAR, reduced ROS production under high-glucose, palmitate or albumin induction. In the present disclosure, adenine was found to reduce ROS production in a dose-dependent manner in HUVEC cell (Table 6), therefore was suggested as a useful treatment to the conditions or diseases which associated with ROS.

### Reference Example: Cancer

Activation of AMPK suppressed COX-2 and mTOR pathways which are important mechanisms of cancer cells growth. Due to the contribution of mTOR and COX-2 to cancer aggressiveness, activation of AMPK is suggested as a rational strategy for cancer therapy. Indeed numerous reports have demonstrated that AMPK activators interrupt cancer progression. For example, phenformin and metformin have been found to inhibit breast tumor development and growth in xenografts mice models. In the present disclosure, adenine was found to inhibit proliferation of human hepatocellular carcinoma cell line Hep G2, human breast adenocarcinoma cell line MCF7 and human colon adenocarcinoma grade II cell line HT29 (example 11). The IC50 of adenine for HepG2, MCF7 and HT29 were 544.1, 537.5 and 531.9 µM, respectively. In HepG2 transplanted mice, chronic treatment with adenine significantly delayed tumor growth in dose-dependent manner. According to present disclosure, the treatment with adenine to activate AMPK activity may prevent or control cancer development and progression.

### Example

Many examples have been used to illustrate the present disclosure. The examples below should not be taken as a limit to the scope of the disclosure.

### Example 1 (Reference)

### AMPK activation assay

Effects of adenine on AMPK activation were evaluated based on the phosphorylation of AMPK protein upon adenine treatment. Mouse muscle cell C2C12, mouse fibroblast 3T3, human liver carcinoma cell Hep G2, human breast adenocarcinoma cell line MCF7 and human colon adenocarcinoma grade II cell line HT29, human umbilical vein endothelial cell HUVEC, Human acute monocytic leukemiacell THP1, human macrophage cell U937, murine microglia cell BV-2, and mouse neuroblastoma cell Neuro2A and dermal papilla cell were cultured in high-glucose Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C, under 5% CO₂. Cells were plated at 3 × 10⁵ per well in 6-well plates. Twenty-four hours after plating, adenine was added to the culture media as indicated. After 30 min cells were lysed and subject to western blot analysis. Equal amount of protein from each sample was separated by SDS-PAGE and then electroblotted on to PVDF membranes. Membranes were blocked with 3% BSA in PBS for 60 min and incubated with an anti-phospho-AMPK (Thr172) antibody (1:2,000, Cell signaling) or an anti-AMPK antibody (1:2,000, Cell signaling) at 4°C for 16 h followed by the corresponding secondary antibody for 1 h at room temperature (RT). Immunoreactive bands were detected by enhanced chemiluminescence and recorded using Kodak film. The detected signals were scanned and then quantified using TotalLab Quant software (TotalLab).

The effect of adenine on AMPK activation is summarized in Table 1. Adenine significantly activated AMPK in all tested cells.

**Table 1**

| Cells | Concentration of adenine (microM) | AMPK activation (fold to control) |
|---|---|---|
| C2C12 | 1 | 1.2 |
| | 10 | 1.7 |
| | 100 | 3.2 |
| | 200 | 3.9 |
| | 600 | 4.1 |
| 3T3 | 1 | 1.1 |
| | 10 | 1.5 |
| | 100 | 2.9 |
| | 200 | 4.0 |
| | 600 | 4.2 |
| Hep G2 | 1 | 1.1 |
| | 10 | 2.1 |
| | 100 | 3.3 |
| | 200 | 3.8 |
| | 600 | 4.2 |
| MCF7 | 1 | 1.2 |
| | 10 | 1.6 |
| | 100 | 2.5 |
| | 200 | 3.4 |
| | 600 | 3.7 |
| HT29 | 1 | 1.1 |
| | 10 | 1.7 |
| | 100 | 2.9 |
| | 200 | 3.4 |
| | 600 | 3.8 |
| HUVEC | 1 | 1.2 |
| | 10 | 1.9 |
| | 100 | 3.2 |
| | 200 | 3.9 |
| | 600 | 4.1 |
| THP1 | 1 | 1.2 |
| | 10 | 2.2 |
| | 100 | 3.7 |
| | 200 | 4.3 |
| | 600 | 4.2 |
| U937 | 1 | 1.1 |
| | 10 | 1.3 |
| | 100 | 2.9 |
| | 200 | 3.7 |
| | 600 | 4.0 |
| BV-2 | 1 | 1.2 |
| | 10 | 1.7 |
| | 40 | 2.6 |
| | 160 | 3.2 |
| Neuro2A | 1 | 1.2 |
| | 10 | 2.1 |
| | 100 | 3.4 |
| Dermal Papilla | 1 | 1.1 |
| | 10 | 1.4 |
| | 100 | 2.1 |
| | 200 | 2.5 |
| | 600 | 2.8 |

### Example 2 (Reference)

### Glucose uptake in vitro

Effects of adenine on glucose uptake were analyzed by measuring the uptake of fluorescent glucose analog (2-NBDG, Molecular Probes) in muscle cell C2C12. C2C12 cells were treated with indicated concentrations of adenine for 30 min at 37°C, and then incubated with 500 µM of fluorescent glucose analog. After 5 min incubation at room temperature, cells were washed three times with Kreb-Hepes buffered solution and fixed in 70% alcohol. The fluorescence of glucose analog in cells was measured using a Fluorescence Microplate Reader System.

The effect of adenine on glucose uptake is summarized in Table 2. Adenine significantly stimulated glucose uptake in C2C12 cells in dose-dependent manner. Data are presented as the mean±SEM of three independent experiments.

**Table 2**

| Agent | Concentration (microM) | Glucose uptake (% to control) |
|---|---|---|
| Adenine | 1 | 117±8.1 |
| | 10 | 261±13.4 |
| | 100 | 315±11.9 |
| | 600 | 338±16.5 |

### Example 3 (Reference)

### Anti-diabetic effects of adenine

To further evaluate the effects of adenine on the modulation of plasma glucose level, the high-fat diet-fed mice were served as a type 2 diabetes animal model. C57BL/6J mice were maintained at 22°C under a 12-h light/dark cycle and fed either a high fat diet (60% kcal% fat) or a normal diet ad libitum. Intraperitoneal injections of adenine (0.1 to 50 mg/kg) or vehicle were given to the high-fat diet-fed mice from the age of 24 weeks and glucose readings were measure at 1 and 3 hr. IP administration of adenine or vehicle only to the high-fat diet-fed mice continued twice a day for 6 days. On day 6, plasma was collected 1 hr after the last dosing for analyzing, evaluating, measuring plasma glucose and triglycerides.

Adenine-injected group showed >30% lower plasma glucose and more than 35% lower plasma triacylglycerides as well as more than 15% lower body weight compare to the control group.

### Example 4 (Reference)

### Adenine suppressed inflammatory response induced by LPS in vitro

Effects of adenine on inflammatory response were evaluated in human THP1 macrophage by examining protein level of intracellular COX-2 and secreted TNFα, IL-1β and IL-6. Differentiation of THP1 monocytes into macrophages was induced by 50 nM PMA for 24 hr. THP1 macrophages were further stimulated by 50 ng LPS for 6 hr in the presence of 10-600 µM of adenine or vehicle followed by cell lysis and western blot analysis. Equal amounts of protein were separated by SDS-PAGE and then electroblotted on to PVDF membranes. Membranes were blocked with 3% BSA in PBS for 60 min and incubated with an anti-COX-2 antibody (1:1,000, Cell signaling), an anti-actin antibody (1:5,000, Cell signaling) at 4° C for 16 h followed by the corresponding secondary antibody for 1 h at room temperature (RT). Immunoreactive bands were detected by enhanced chemiluminescence and recorded using Kodak film. The detected signals were scanned and then quantified using TotalLab Quant software (TotalLab). The secreted TNFα, IL-1β and IL-6 were analyzed by enzyme-linked immunosorbent assays.

The effect of adenine on immune response is summarized in Table 3. The expression of COX-2 and secretion level of TNFα, IL-1β and IL-6 were significantly reduced in adenine treated macrophages compared with control cells.

**Table 3**

| Adenine (microM) | TNFα (% to control) | IL-1β □ (%to control) | IL-6 (% to control) | COX-2 (% to control) |
|---|---|---|---|---|
| 0 | 100±4.7 | 100±11.3 | 100±8.5 | 100±2.9 |
| 10 | 85±9.1 | 91±8.4 | 88±6.3 | 81±4.4 |
| 100 | 41±2.6 | 29±5.5 | 21±7.8 | 59±3.5 |
| 600 | 23±1.8 | 17±3.7 | 14±6.2 | 38±5.3 |

### Example 5 (Reference)

### Adenine suppressed 2,4,6-trinitrobenzene sulfonic acid (TNBS) induced inflammation in vivo

To further evaluate the effects of adenine on inflammatory response, TNBS-induced inflammatory bowel disease (IBD) mice model was used. C57BL/6J mice were maintained at 22° C, under a 12-h light/dark cycle. Relapsing colitis was induced with five escalating doses of TNBS, 0.5 mg, 0.75 mg, 1.0 mg, 1.25 mg, and 1.5 mg, in 50% ethanol were administered respectively for 0.1 mL per mouse weekly. After the third administration of TNBS, daily intraperitoneal injection of adenine (0.01, 0.1, 5 or 30 mg/kg body weight) or saline were given to mice. Two days after the fifth TNBS administration, mice were sacrificed. The inflammatory cytokines including TNF, INFγ and IL-17 from colonic lysates were evaluated by enzyme-linked immunosorbent assays.

The level of TNF, INFγ and IL-17 were significantly reduced in colon of adenine treated mice in dose-dependent manner compared with non-adenine treated mice. In addition, treatment of adenine also rescued the body weight loss caused by TNBS.

### Example 6 (Reference)

### Amyloid β peptide and autophagy assay

Effects of adenine on Amyloid β peptide were analyzed in mouse neuroblastoma cell Neuro2A. Neuro2A cells were cultured in high-glucose Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C under 5% CO₂. Cells were plated at 3 × 10⁵ per well (6-well plate). 24 h after plating, cells were transfected with human APP695 and treated with indicated concentration of adenine for 24 h followed by cell lysis and western blot analysis. Equal amounts of protein were separated by SDS-PAGE and then electroblotted on to PVDF membranes. Membranes were blocked with 3% BSA in PBS for 60 min and incubated with an anti-Aβ antibody (1:1,000, abcam), an anti-LC3 antibody (1:1,000, Cell signaling), and an anti-actin antibody (1:5,000, Cell signaling) at 4°C for 16 h followed by the corresponding secondary antibody for 1 h at room temperature (RT). Immunoreactive bands were detected by enhanced chemiluminescence and recorded using Kodakfilm. The detected signals were scanned and then quantified by using TotalLab Quant software (TotalLab).

The effects of adenine on sAβ production and LC3-II/LC3-I ratio are summarized in Table 4. Adenine significantly reduced Aβ amount and increased LC3-II/LC3-I ratio in dose-dependent manner in Neuro2A cells. Because the conversion of LC3-I to LC3-II is indicative of autophagy activity, the higher LC3-II/LC3-I ratio in adenine treated cells reflects the ability of adenine to induce autophagy activity.

**Table 4**

| Adenine (microM) | sAβ level (% of control) | LC3-II/LC3-I ratio (relative to control) |
|---|---|---|
| 0 | 100 ± 6.1 | 1.0 ± 0.1 |
| 10 | 89 ± 7.5 | 1.2 ± 0.1 |
| 20 | 63 ± 2.2 | 1.8 ± 0.3 |
| 30 | 48.1 ± 1.7 | 2.8 ± 0.2 |
| 40 | 31.7 ± 5.1 | 2.9 ± 0.2 |
| 50 | 29.4 ± 3.6 | 3.2 ± 0.3 |

### Example 7 (Reference)

### Adenine rescued neurodegeneration of Aβ-Induced Alzheimer's disease model mice

Aβ25-35 was purchased from Sigma-Aldrich (St. Louis, Missouri). The peptides were dissolved in distilled saline and aggregated by incubation at 37°C for 7 days before injection. C57BL/6J mice were maintained at 22° C under a 12-h light/dark cycle. Adult mice were anesthetized by ketamine (500 mg/kg) and xylazine (100 mg/kg) and placed in a stereotaxic frame. Five nmol of the aggregated Aβ25-35 were injected into the lateral ventricle using a 10 µl Hamilton syringe. The target anterior-posterior (AP), medial-lateral (ML) and dorsoventral coordinates were -0.5 mm, ±1 mm and -2.5 mm relative to the bregma. To evaluate the effect of adenine on neurodegenerative disease, Aβ infusion mice were daily intraperitoneally injected with 0.01, 0.1, 5 or 30 mg/kg bodyweight of adenine or vehicle for 4 weeks. The cognitive functions of these mice were analyzed by using Morris water maze assay after 4 weeks injection. The water maze was performed in a circular pool filled with water and a platform was submerged below the water's surface in the target quadrant for hidden platform test. During the 5-day hidden platform test, mice were randomly placed into starting points of the pool in each daily trial (6 trials per day). The probe trial was performed 1 day after 5-day hidden platform test. For the probe trial, the platform used in the hidden platform test was removed and the starting point was in the quadrant opposite the target quadrant. Mice were allowed to swim in the maze for 60 s and recorded by a video camera. The latency to find the platform and swim paths was analyzed by software.

In the hidden platform test, adenine treated AD mice took significantly shorter time to find platform in dose-dependent manner than control AD mice. This result demonstrated adenine treatment rescued the impairments of special learning and memory of AD mice. Further, adenine treated AD mice spent higher percentage time in target quadrant in probe assay than control AD mice, which indicated that adenine improve the retention of memory.

### Example 8

### Adenine inhibited fibroblast proliferation

Human fibro blast cell line 3T3 were cultured in high-glucose Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C under 5% CO₂. For cell proliferation assay, 3T3 cells were plated at 1 × 10⁵ per well (6-well plate). Twenty-four hours after plating, cells were treated with indicated concentration of adenine for 72 h and the number of viable cells was counted. Cells were detached using trypsin-EDTA solution and stained with trypan blue. The living cells were counted using hemocytometer.

The effects of adenine on 3T3 cell proliferation are summarized in Table 5. According to Table 5, adenine significantly inhibited 3T3 cell proliferation in dose-dependent manner. Data are presented as the mean±SEM of three independent experiments.

**Table 5**

| Adenine (microM) | Cellnumber (% to control) |
|---|---|
| 0 | 100±4.3 |
| 10 | 91±2.7 |
| 50 | 73±8.1 |
| 100 | 64±5.3 |
| 200 | 48±2.8 |
| 500 | 33±6.4 |
| 1000 | 27±11.3 |

### Example 9

### Adenine enhances wound healing and reduces scar formation

C57BL/6J mice were maintained at 22°C under a 12-h light/dark cycle. The experiments were performed with 12-week-old mice. After anesthetized by an intraperitoneal injection of ketamine (500 mg/kg) and xylazine (100 mg/kg), 6-mm full-thickness excisional skin wounds was made on the backs of mice using 6-mm skin biopsy punches. Immediately after wounding, 10-1200 µM of adenine or saline alone was applied to the wound bed. The skin wounds were then covered by semipermeable transparent dressing and fixed to the skin. The mice were treated with adenine or vehicle for 14 days and then sacrificed. The scar formation is assessed by Masson's trichrome staining (the tissue was fixed by 4% paraformaldehyde).

After 14 days of treatment, the extent of closure was significantly greater in adenine treated mice in dose-dependent manner than in control mice. According to histological examination of the regenerated tissue, topical treatment with adenine significantly decreased the scar width compared to vehicle treated wounds.

### Example 10 (Reference)

### Adenine reduced ROS production

Human umbilical vein endothelial cells (HUVECs) were cultured in Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C under 5% CO₂. Cells were plated at 2×10⁴ per well in black 96-well. Twenty-four hours after plating, medium was changed to fresh DMEM containing either 5.6 or 30 mM glucose and treated with indicated concentration of adenine. Twenty-four hours after treatment, the intracellular ROS was detected using H2DCF-DA. Cells were washed once with PBS and then incubated with 100 µM DCF at 37°C for 30 min. Then the fluorescence of DCF was measured using a Fluorescence Microplate Reader System at 485-nm excitation and 530-nm emission wavelengths.

The effect of adenine on ROS production is summarized in Table 6. Adenine significantly reduced hyperglycemia-induced ROS production in dose-dependent manner.

**Table 6**

| Adenine (microM) | Glucose (mM) | ROS production (% of 5.6 mM glucose) |
|---|---|---|
| 0 | 30 | 275 ± 8.1 |
| 10 | 30 | 211 ± 4.3 |
| 100 | 30 | 116 ± 1.7 |
| 200 | 30 | 38.1 ± 2.9 |
| 600 | 30 | 21.7 ± 3.1 |
| 1200 | 30 | 22.4 ± 2.5 |

### Example 11 (Reference)

### Cancer cell growth inhibition assay

Human liver hepatocellular carcinoma cell line Hep G2, human breast adenocarcinoma cell line MCF7 and human colon adenocarcinoma grade II cell line HT29 were used to evaluate the effects of adenine on cell proliferation. Those cell lines were obtained from ATCC and were cultured in high-glucose Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C under 5% CO₂. Cells were plated at 1 × 10⁵ per well (6-well plate). Twenty-four hours after plating, cells were treated with indicated concentration of adenine for 72 h and then followed by cell counting. Cells were detached using trypsin-EDTA solution and stained with trypan blue. The living cells were counted using hemocytometer.

The IC50 of adenine for HepG2, MCF7 and HT29 were 544.1, 537.5 and 531.9 µM, respectively.

### Example 12 (Reference)

### Tumor growth assay

Human liver hepatocellular carcinoma cell line Hep G2 were cultured in high-glucose Dulbecco's modified Eagle's medium (DMEM) containing 10% fetal bovine serum (FBS), 4 mM L-glutamine, 2 mM sodium pyruvate and 1% penicillin/streptomycin (Invitrogen GibcoBRL, Carlsbad, Calif., USA) at 37°C under 5% CO₂. For tumor implantation, 5 × 10⁶ Hep G2 cells were injected subcutaneously into 8-week-old male nonobese diabetic-severe combined immunodeficiency (NOD-SCID) mice. After implantation, the mice were daily intraperitoneally injected with 5, 20 or 50 mg/kg body weight of adenine and the tumor size was monitored every 3 days. The growth of tumor was significantly retarded in adenine treated mice compared with control mice 14 days post implantation.

The scope of the present disclosure is defined only by the appended claims.

## Claims

1. A composition for use in preventing scar formation during wound healing, **characterized in that** the composition comprises adenine and/or a pharmaceutically acceptable salt thereof as a sole active ingredient effective on preventing scar formation during wound healing, wherein the adenine and/or the pharmaceutically acceptable salt thereof is administrated to a mammal in need of such treatment.

2. A composition for use in enhancing wound healing, **characterized in that** the composition comprises adenine and/or a pharmaceutically acceptable salt thereof as a sole active ingredient effective on enhancing wound healing, wherein the adenine and/or the pharmaceutically acceptable salt thereof is administrated to a mammal in need of such treatment.

## Patentansprüche

1. Zusammensetzung zur Verwendung beim Verhindern von Narbenbildung während der Wundheilung, **dadurch gekennzeichnet, dass** die Zusammensetzung Adenin und/oder ein pharmazeutisch zulässiges Salz davon als einzigen zum Verhindern von Narbenbildung während der Wundheilung wirksamen Wirkstoff umfasst, wobei das Adenin und/oder das pharmazeutisch zulässige Salz davon einem Säugetier verabreicht wird, das einer solchen Behandlung bedarf.

2. Zusammensetzung zur Verwendung beim Verbessern der Wundheilung, **dadurch gekennzeichnet, dass** die Zusammensetzung Adenin und/oder ein pharmazeutisch zulässiges Salz davon als einzigen zum Verbessern der Wundheilung wirksamen Wirkstoff umfasst, wobei das Adenin und/oder das pharmazeutisch zulässige Salz davon einem Säugetier verabreicht wird, das einer solchen Behandlung bedarf.

## Revendications

1. Composition destinée à être utilisée pour prévenir la formation de cicatrices durant la cicatrisation, **caractérisée en ce que** la composition comprend de l'adénine et/ou un sel pharmaceutiquement acceptable de celle-ci comme seul ingrédient actif efficace pour prévenir la formation de cicatrices durant la cicatrisation, dans laquelle l'adénine et/ou le sel pharmaceutiquement acceptable de celle-ci est administré à un mammifère ayant besoin d'un tel traitement.

2. Composition destinée à être utilisée pour améliorer la cicatrisation, **caractérisée en ce que** la composition comprend de l'adénine et/ou un sel pharmaceutiquement acceptable de celle-ci comme seul ingrédient actif efficace pour améliorer la cicatrisation, dans laquelle l'adénine et/ou le sel pharmaceutiquement acceptable de celle-ci est administré à un mammifère ayant besoin d'un tel traitement.
